# EUROPEAN PATENT SPECIFICATION

(11) **EP 2 814 377 B1**
(45) Date of publication and mention of the grant of the patent: **04.03.2026**
(21) Application number: 13712330.3
(22) Date of filing: 24.01.2013
(51) Int. Cl.: A61B 3/10, G01B 9/02

(54) **FAST MEASUREMENT OF OCULAR AXIAL LENGTH**
SCHNELLE MESSUNG EINER OKULARACHSENLÄNGE
MESURE RAPIDE DE LA LONGUEUR AXIALE DE L'OEIL

(30) Priority: 24.01.2012 IT PI20120009
(43) Date of publication of application: 24.12.2014
(73) Proprietor: VISIA Imaging S.r.l., 52027 San Giovanni Valdarno (AR) (IT)
(72) Inventor: FOGGI, Alessandro, 52027 San Giovanni Valdarno (AR) (IT); PEZZATI, Luca, 50134 Firenze (FI) (IT)
(74) Representative: Emmi, Mario
(86) International application number: PCT/IB2013/050621
(87) International publication number: WO 2013/111090

(56) References cited:
- EP-A1- 0 956 809
- EP-A1- 0 956 809
- EP-A1- 2 395 343
- EP-A1- 2 395 343
- US-A1- 2005 140 981
- US-A1- 2005 140 981
- US-A1- 2011 109 913
- US-A1- 2011 109 913

## Description

### Technical field

The present invention refers to the technical field relative to optical measuring instruments, in particular ophthalmology instruments with which the parameters necessary for designing intraocular lenses to be installed in cataract surgeries are detected.

In particular, the invention refers to an innovative reflecting element to be used in Michelson-type interferometers.

### Background art

More or less complex machineries for measuring parameters for designing intraocular lenses have long been known.

Such machineries permit the calculation of the following essential parameters for designing an ocular lens:
1) Keratometry, which is the measuring of the three sizes Rf, Rs, Δk. This measure serves to evaluate the shape of the cornea through the detection of the parameters Rf and Rs (flatter meridian radius and curver meridian radius) and of the parameter Δk that represents the difference between the said two radiuses.
2) Anterior chamber central depth (ACD): this parameter measures the intraocular distance between the posterior face of the cornea and the anterior face of the crystalline, which is generally of the order of about 2÷4mm.
3) Axial length (AL): this third parameter, instead, measures the intraocular axial distance between the external surface of the cornea and the retina, which average distance is of about 25mm.

The exact knowledge of these parameters helps designing an intraocular lens that better interprets the refractive features of each patient.

The known machineries generally integrate among them various parts, each one of which is appointed to evaluate one of the parameters listed above.

In particular, such machineries provide a support frame on which the measuring instrument is arranged and a support on which to place the chin and the upper face of the patient, so as to allow a comfortable positioning of the eye with respect to the aiming system.

One of the known principles on which the measuring of the axial length is based is the low-coherence interferometry, and in particular the well-known Michelson interferometer is used in its configuration in optical fiber.

The Michelson interferometer in optical fiber is composed of four branches of optical fiber crossed by coherent or incoherent electromagnetic (e.m.) radiation. The four branches depart from a central node called optical fibers coupler. An inlet branch is connected to a light source coupled in fiber. Such a source is a super-luminescent diode (SLED) that emits radiation around a wave length of 820 nm. The radiation emitted by the SLED is directed to the coupler, where it undergoes a division into two opposed branches: about 10% of the energy enters the low-intensity branch, while the 90% enters the other branch. The radiation outgoing from the low-intensity branch is collimated by a lens and directed to the eye of the patient, while the radiation outgoing from the other branch is collimated by a second lens and directed to the reference surface (mobile) of the interferometer. This one is generally a low-reflectance element (about 4%) constituted of a single flat plate of optical glass (or another optical material) translatable along a guide parallel to the axis of the collimated beam exiting from the fiber.

The radiation reflected by the ocular surfaces of the patient, in good alignment conditions, re-enters the fiber back-crossing the lens of collimation of the low-intensity branch. The radiation reflected by the reference surface also re-enters the other branch crossing the second lens. The two beams of re-enter crossing the coupler are gathered in this way: 90% of the energy coming from the eye is gathered with 10% of the energy coming from the reference surface. This radiation, present in the fourth branch of the interferometer, is directed to a photodiode that measures its intensity, in its turn connected to an amplifier, to an analogical/digital converter (ADC) and to an electronic processor.

The collimator that aims at the eye is fixed at a pre-determined distance in such a way that the two arms of the interferometer have an almost equal difference of optical path, the arm of the plate being shorter by a few millimeters.

Both the ocular surfaces and the plate reflect back certain quantities of radiation that, through the two collimators, re-enter the optical fibers and by recombining through the central node they are directed to the photodiode. During the measuring the plate translates in such a way as to reach the position in which its optical distance from the source is equal to that of the external surface of the eye. In this condition, the interferometer has equal arms and produces interference with any type of radiation by which it is illuminated (coherent or incoherent). In this first position, in particular, the two waves reflected (the one of the external surface of the cornea and the one of the plate) overlap in constructive interference, generating a maximum of signal.

Going on with the translation, other maxima of the signal are obtained in all those positions in which the optical distance between the reference surface and one of the intraocular surfaces that are the object of the measuring is identical. The temporal tracing of the signal obtained by the interferometer during the translation of the reference plate thus contains a series of maxima, in correspondence of all the axial positions of the intraocular surfaces to measure. In particular, there are two intense maxima in correspondence of the anterior surface of the cornea and of the surface of the retina. All the maxima are surrounded by an envelope having an oscillating shape and caused by secondary minima and maxima of the interference, due to the partial coherence of the light emitted by the SLED. The secondary maxima, in the case in question, are about a hundred for each main maximum. By analyzing the envelope with appropriate known mathematical techniques, the position of the main peak is obtained. Last, by knowing the scanning speed, or alternatively the position of each point of the temporal tracing (for example, through an appropriate sampling, executed by using the signal of an encoder) the optical distance of the main peaks can be deduced, from which the geometric distance of the axial length (AL) sought is obtained.

A problem of the measuring with this technology resides in the fact that the distance that the reference plate has to cover to measure the axial length also of eyes bigger than the average ones is relatively long, for example of the order of 45mm. During the scanning time, in which the plate covers said distance, it is easy that, in a way unnoticed, the patient rotates or more generically moves the eye with a sudden and uncontrolled movement. This movement of the eye during the measuring causes the registration of incorrect distances between the intraocular surfaces measured.

It is to be highlighted that a simple solution to said technical problem that foresees an increase in the scanning speed is not viable since there would be problems of detection of a very-high-frequency signal. The signal that increases in frequency is the carrier of the envelope due to the alternation of clear and dark fringes in the profile.

Therefore, there is actually the need of a device, in particular of a reflecting arrangement, which results configured in such a way as to reduce as much as possible, preferably to halve, the travel time currently required without increasing the scanning speed.

A solution, for example, has been proposed in US patent application no. US2005/0140981 filed on 30 June 2005. This application addresses the technical problem of how to obtain an equipment for ophthalmic measurements that is versatile and that does not require the arrangement of an excessive number of components placed in front of the patient. The solution is obtained with a modular apparatus in which the various components are placed at a distance and are among them connectable and detachable. One of these components also provides a Michelson interferometer comprising an arrangement on which two reflecting plates are arranged, one in front of the other and adjustable each time at distances different from each other. On said two plates the beam of light coming from a branch of the interferometer is sent, while a second beam of light, through two lenses, is directed to the two optical surfaces of which the distance wants to be measured. The two reflecting plates have to be each time adjusted at a reciprocal distance (distance d2) which more or less coincides with the distance that it is expected to be measured (in the example given in paragraph [0070] an optical distance of 34mm is indicated).

In this way, it is as if there were two interferometers in parallel and therefore it is not necessary that the single plate makes a long path to intercept the two surfaces to be measured as per the background art. The method provides an initial adjustment of the distance between the plates coinciding with the distance that is expected to be found to then make a high-frequency oscillation of the plates in such a way as to obtain a measuring distance at the end of the oscillation.

The arrangement is therefore made to oscillate at a frequency of about 10 Hz in such a way that each plate oscillates around the surface of which the measuring wants to be made. During the oscillation every time that the optical path between the light reflected by the first plate and the light that comes from the first optical surface is the same there is an interference peak. The same thing takes place when the optical path between the reflected light coming from the second plate and the second optical surface is the same. An envelope of peaks is thus created, which allows somehow to obtain the ocular distance that wants to be measured.

It is obvious that this solution has anyway significant technical inconveniences.

The first among all of them is that it is necessary, every time, to adjust the two plates at a relative distance that more or less coincides with the intraocular distance that is expected to be found in the measuring. This is so because the method provides an oscillation around the surface that wants to be measured with the obvious need to have to adjust every time a new distance between the plates on the basis of the distance that is expected to be found.

It is obvious, however, that a manual adjustment, every time, is not a comfortable operation and it affects in a significant way the precision of the measuring. The human eye in fact provides intraocular distances that are very different from subject to subject. Although a sort of "average" of the distances can exist, in a pre-determined subject a certain intraocular distance can be very different with respect to the one of another patient (for example, if the patient is an adult or a child). This means that the initial adjustment between the two plates on the basis of the distance that is expected to be measured is an operation that inevitably takes to an erroneous measuring with the risk of making an oscillation that will never intercept the optical surface. If it is expected to measure a pre-determined distance d1 and then in the patient his actual distance is very different from the one that was expected, the standardized adjustment of the plates (distance d2) can result absolutely inappropriate for that patient. In this case, the oscillation of the plates could not intercept any optical surface or even intercept optical surfaces different from the ones that want to be measured, thus obtaining an absolutely erroneous result.

Therefore, the use of an oscillation with a manual adjustment of the distances between the plates on the basis of the distance that is expected to be found affects the precision of the measuring.

Moreover, the method described in US2005/0140981 requires a high-frequency oscillation for a relatively long time, and, during this time, the patient could move the eye, generating a further error in the measuring.

### Summary of the invention

It is therefore the aim of the present invention to provide a new interferometric device of the "Michelson" type that allows to solve, at least in part, said technical inconveniences.

In particular, it is the aim of the present invention to provide a new interferometric device in which the motion required to the reflecting element or arrangement results significantly reduced (even halved) with respect to the one of the background art, though resulting particularly simple from the point of view of the structure and allowing at the same time not to have to increase the scanning speed beyond the practical limits.

It is therefore the aim of the present invention to provide a new interferometric device, and relative method, in which the measuring obtained does not require high-frequency oscillations and manual adjustments of the distances between the plates on the basis of the distances to be measured, thus resulting very precise; the reduction of the time necessary to make it is also obtained.

In particular, it is the aim of the present invention to provide a new interferometric device, and relative method, in which there is not the risk, during the measuring, of not intercepting any optical surface, or even erroneous optical surfaces with respect to those that are expected to be measured.

These and other aims are therefore reached with the present interferometric device for measuring an axial length (AL) as per claim 1.

The interferometer that is the object of the invention, comprising an element (7) at least partially reflecting and translatable along a motion direction in such a way that it can move apart or come near with respect to an emitting light source (5). Said element (7) thus provides at least a first surface (8) and at least a second surface (9), which are both at least partially reflecting. The two surfaces are arranged at a pre-determined reciprocal distance **(d).**

Moreover, the two surfaces (8, 9) are arranged in the arrangement (7) in such a way as to keep said reciprocal distance **(d)** constant at least during the translation of the arrangement (7) in the scanning phase.

In accordance with the invention, a motorized guide system (500) is provided to command the movement of the reflecting arrangement (7) which is controlled in such a way that the scanning is completed in a single motion of translation in a direction starting from an initial position in which the first surface (8) generates the first interference peak at the beginning of the translation and with such a fixed distance **(d)** between the plates so that afterwards during said translation the second plate (9) generates the second interference peak.

Unlike the background art described, therefore, no oscillation is carried out around the optical surfaces in order to complete the scanning but instead a single motion of translation is made starting from a starting point in which the first plate immediately generates interference. The distance between the plates is fixed and is such that afterwards, during the translation, the second interference peak is generated. Such a run is enough for intercepting the two optical surfaces of interest and thus generate the interference peaks that allow to obtain to the axial length (AL).

By single run of translation in a direction it is meant not a high-frequency oscillation but a single forward run. The fact remains that, in order to optimize the measuring, it is possible to carry out two or three runs, (for example three forwards and three backwards). The measuring is completed already at the first forward run but the measurings obtained in the remaining runs can be used to obtain the verification of the calculation found or an envelope that optimizes the calculation. Nevertheless, regular high-frequency oscillations are not made.

In this way, the measuring is quick and precise. There is not the risk of not intercepting any surface and it is not required to modify the distance between the plates for different measures.

During the translation of the entire element (7) to carry out the measuring, the posterior reflecting element (9) reaches almost immediately the position in which its distance from the source is equal to the one of the retina going to constructive interference and producing the peak that measures the sought distance with respect to the peak generated by the anterior surface (8) when the cornea is reached.

This solution is as if a simultaneous use of two separate interferometers was actually allowed and of which the "outputs" are however combined in a unique tracing containing the envelopes relative to all the peaks generated by the reset of the difference of optical path of the first (8) and of the second (9) surface during the scanning.

The first surface (8), as soon as it intercepts the cornea, generates a first peak, while the second surface, in virtue of the distance **(d)** at which it is positioned with respect to the first one (8), immediately after intercepts the retina, generating the second peak. It is clear that the detection of the two peaks, used in a specific formula to obtain the axial distance sought, takes place in accordance with said solution in half the time with respect to the background art described.

Such a solution is therefore structurally simple and economical and allows to reduce significantly or even to halve the scanning distance covered by the reference surface, the whole at the same scanning speed. The time necessary to operate the measuring is therefore reduced significantly, reducing to the minimum the risk of accidental movement of the eye of the patient during the measuring.

According to the invention, the first (8) and the second surface (9) are placed parallel and coaxial between them.

Advantageously, there can be provided adjusting means for allowing a reciprocal sliding of the two surfaces (8, 9) in the arrangement (7) in such a way as to adjust their reciprocal distance **(d),** and fixing means to block said reciprocal distance or, alternatively, be directly fixed.

According to the invention, said reflecting arrangement (7) has an optical length comprised between 15mm and 25mm.

According to the invention, the distance between the first (8) and the second surface (9) is comprised between 12mm and 19mm.

Advantageously, the motorized system (500) is connected to an electronic processor (PC) that commands the movement between two extreme positions.

Advantageously, the following are further provided:
- A system of acquisition/analysis of the images (300, 400) configured to acquire a plurality of images at different focal distances and select the image that results in focus;
- A translation system (210) that allows to translate the interferometer (1) as a function of the images acquired in such a way that when the image acquired results in focus the interferometer results positioned with the first surface (8) substantially at an interference distance with respect to the first surface (100) of the axial length (AL) to be detected.

Advantageously, a frame is provided on which said acquisition/analysis system of the images and the interferometer (1) are provided, said frame being reciprocable in such a way as to allow the acquisition of images at different focal distances.

It is also here described a method for measuring the intraocular axial length (AL) by means of a Michelson-type interferometer (1) comprising an arrangement (7) at least partially reflecting the light and provided with at least a first (8) and with at least a second (9) surface at least partially reflecting, arranged between them at a pre-determined reciprocal distance (d), said method comprising the following operations:
- Initial positioning of the reflecting arrangement (7) in such a way that the first reflecting surface (8) is found at a distance (d") from the collimator (5) inferior to the distance (d') of the collimator (4) from the cornea (100) of the eye;
- Sending of the light beam through the two branches (4', 5') terminating in the two collimators (4, 5);
- Translation of the reflecting element operating a single translation run along a measuring direction in such a way as to obtain the generation of two interference peaks (30, 40) in correspondence of the reaching of the two interference positions in which respectively the optical distance of the first surface (8) from the source (5) is equal to that of the external surface of the eye (100) with the source (4) and the optical distance of the second surface (9) from the source (5) is equal to that of the source (4) from the retina;
- And wherein the said two surfaces (8, 9) are arranged in the arrangement (7) in such a way as to keep said reciprocal distance **(d)** constant at least during the translation of the arrangement (7) in the scanning phase and so that the first surface (8) generates the first interference peak at the beginning of the translation and afterwards during said translation the second plate (9) generates the second interference peak.

Advantageously, an operation of acquisition of a plurality of images at different focal distances is preliminarily provided, with a consequent translation of the interferometer as a function of the optimal focal distance obtained so that when the image acquired results in focus the interferometer results positioned at a distance in which the first surface (8) substantially generates interference immediately with respect to the first surface (100) of the eye.

Advantageously, the distance between the first and the second surface (8, 9) is fixed and is not modified when the measurings to be carried out of the intraocular distances vary.

According to the invention, the distance between the first (8) and the second surface (9) is comprised between 12mm and 19mm.

### Brief description of drawings

Further features and advantages of the present device and relative method, in accordance with the invention, will result clearer with the description that follows of some preferred embodiments, made to illustrate but not to limit, with reference to the annexed drawings, wherein:
- Figure 1 and figure 2 show a schematization of the Michelson interferometer in accordance with the present invention;
- Figure 3 schematizes the detecting phase of the first peak;
- Figure 4 schematizes the detecting phase of the second peak;
- Figure 5 shows schematically the overall graph with the relative peaks (double plate) and, just as a way of example, shows how the graph is a sort of overlapping between two interferometers that work independently and simultaneously;
- Figure 6 schematizes graphically the calculation that allows to obtain the axial distance sought;
- Figure 7 shows a possible technical solution for the movement of the arrangement 7 provided with the two plates (8, 9);
- Figure 8 shows the frame on which the interferometer is arranged and its translation in order to obtain an image of the eye that results in focus.

### Description of some preferred embodiments

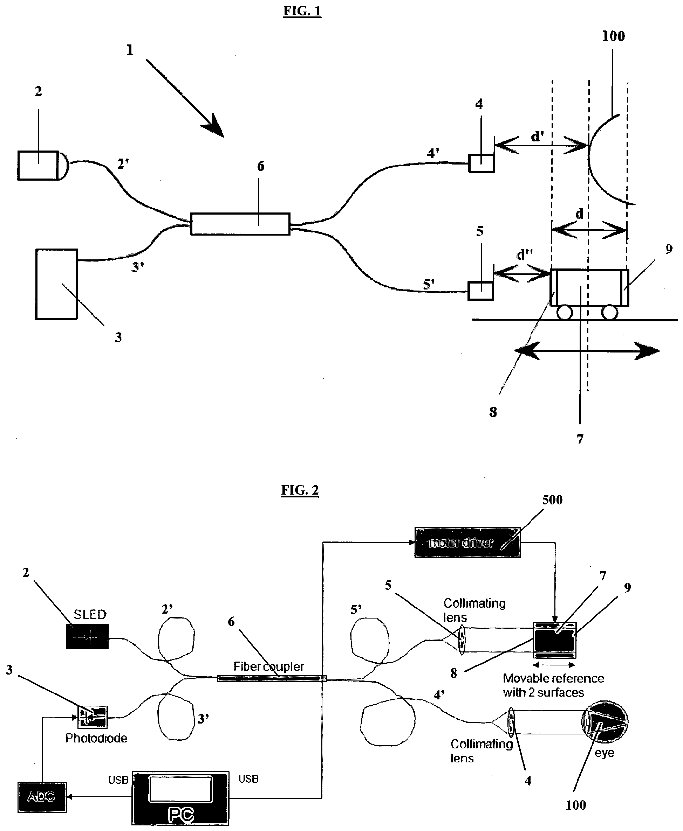

With reference to figure 1 and to figure 2, an interferometer 1 is described in accordance with the present invention.

The interferometer, as per the background art, provides a coupler 6 ("fiber coupler" as per figure 2) to which the four branches of optical fiber converge (2', 3', 4', 5'). A first branch 2' is connected to a source 2 (SLED in figure 2) and a second branch 3' is connected to the receiving photodiode 3 (Photodiode 3 in figure 2) that elaborates in return the reflected light beam. On the opposite part to the node 6 the other two branches of optical fiber (4', 5') converge and of which one (the branch 4') connected to the collimator 4 placed opposite the eye 100 and the other one (the branch 5') connected to a collimator 5 placed opposite a reflecting element 7.

Figure 2, as already described in the preamble of the background art, also describes the electronic processor (PC) which is placed in communication with the photodiode 3 through a converter (ADC).

As per the state of the art, therefore, the beam emitted by the source 2 is sent in part to the eye 100 and in part to the reflecting element 7 through the two ramifications (4', 5') in such a way that the photodiode 3, in return, receives the beam reflected through the branch 3' and analyzes the interference between said reflected waves.

In accordance with the invention, the reflecting element 7 (also called reflecting arrangement 7) provides a first plate 8 and a second plate 9 at least partially reflecting between them, parallel and distanced by a pre-determined fixed quantity **(d).** The plates are preferably flat.

In the configuration of the invention the reflecting element 7 has the shape of a cylinder which anterior face provides the first reflecting plate 8, while the posterior face constitutes the second reflecting plate, which are coaxial with each other (apart from being, as already said, parallel).

The cylinder 7, as schematically shown in figure 1 and in figure 2, is therefore translatable along a sliding binary and in accordance with the double direction of the arrow always shown in figure 1 and in figure 2. In this way, the translation of the cylinder causes an integral translation of the two reflecting faces (8, 9) which, belonging to the cylinder and being fixed to it, keep their reciprocal distance **(d)** unvaried during all the translation.

Always figure 2 also shows a guide engine 500 (motor driver of figure 2) which is activated by the PC and controls the movement of the cylinder.

The cylinder 7, for the purposes of the following invention, has lengths comprised within a range between 12mm and 19mm and, preferably, a length of about 12,5mm in such a way as to ensure the maxima decoupling between the positions where the maxima measured of cornea and retina are, in normal conditions, diminishing.

It is reminded that, actually, the distances mentioned here are the geometricals of the cylinder and therefore, as the preferred embodiment of the invention provide the use of a standard glass (BK7, with a refraction index n=1.5), the optical distances are those geometrical multiplied by said factor n of 1.5. Basically, the optical length is given by the product of the geometrical length with refraction index of the material measured at 820nm. With the values of the geometrical length of the cylinder within said ranges, a value of optical length of the optimal cylinder comprised between 15mm and 21mm can be extrapolated.

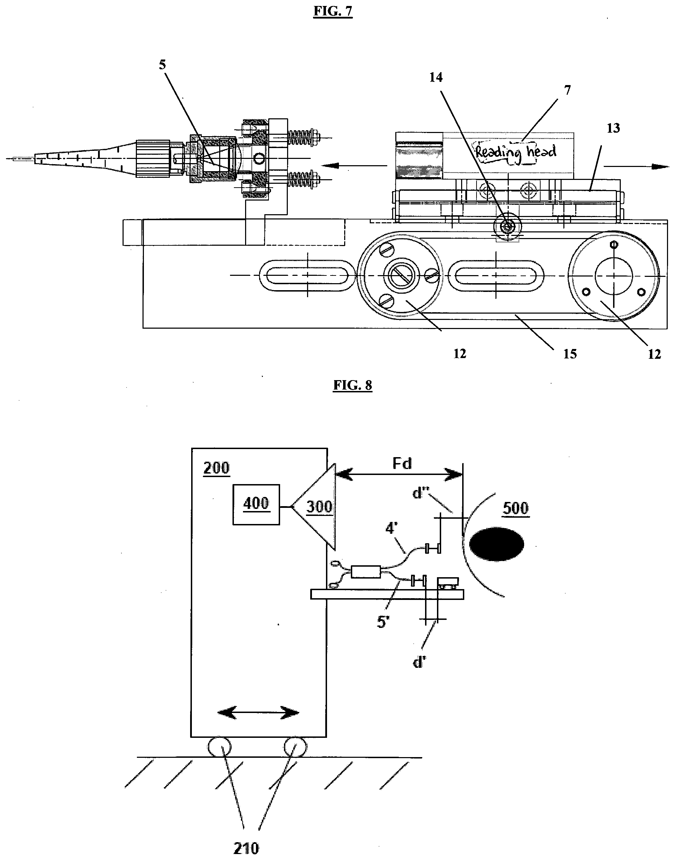

Figure 7 shows structurally a constructive solution adopted to allow the translation of the arrangement 7. The solution shows two wheels 12 that are rotatable around their hinge axis and connected by a dragging element, for example a belt 15. A motorized system, for example electric, is used for commanding the rotation of one or both wheels 12, therefore causing the dragging in motion of the belt 15. A grasping element 14, for example a pair of opposed dies, a plier or a pivot that is inserted in the belt, is integral to the slide 13 and is interposed between the slide 13 and the belt 15. The element 14 grasps the belt in such a way as to allow that the belt drags the slide during its alternate motion. As shown in figure 7, the two directions of alternate motion from the arrangement 7 are highlighted.

In a possible variant, it would be possible to foresee that the arrangement 7, for example in the shape of a cylinder of other shape, provides a system of adjustment of the reciprocal distance (d) between the two plates (8, 9). For example, a simple sliding system, assembling the two plates on appropriate binaries within the cylinder 7, would allow to adjust their reciprocal distance (d). A blocking system then allows to fix the selected position so as to conduct the scanning with said fixed distance (d).

This solution, although structurally more complex, allows to adapt the arrangement 7 to particular biometric characteristics of the patient under examination, for example in the case of children in which the distances to be measured could be different from those of an adult.

Although in all the embodiments described it has been indicated that the two plates are coaxial between them, actually nothing would impede to arrange them in a non-coaxial way. It is in fact enough that the beam entering the first plate intercepts also the second plate without they being obligatorily perfectly coaxial.

A further non-claimed variant could even foresee that the twc plates are arranged in such a way as not to result either coaxial or parallel between them. This solution would be possible arranging, for example, the second plate at a right angle with respect to the first one, therefore forming a corner. In this case, it would be enough to arrange a reflecting element in such a way that the last one reflects the entering beam through the first plate on the second one placed at a right angle with respect to the first one.

Moreover, it is clear that equivalent solutions not according to the invention can anyway provide different shapes of the reflecting element, for example not a cylinder but a parallelogram.

In use, therefore, the functioning is the following.

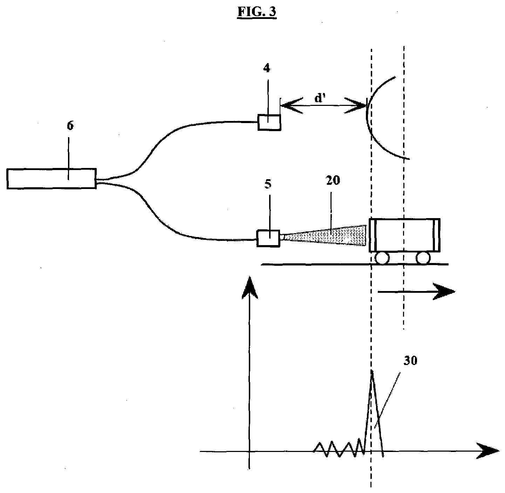

Initially, figure 1 shows an initial position in which the cylinder 7 is placed at such a distance from the collimator 5 that the first plate 8 results at a distance (**d"**) inferior to the distance **(d')** between the collimator 4 and the surface 100 of the eye. In this way, on the basis of the appropriate geometrical length of the cylinder, it is as if virtually the cylinder 7 resulted astride the external surface of the eye, that is the surface of the vitreous *humor* 100. By the term "astride" it is intended that the first plate is placed at an optical distance inferior with respect to the surface of the cornea, while the second plate is at an optical distance superior to the cornea but inferior to the retina. The first reflecting surface 8 will therefore be found in a backward position with respect to the eye 100 exactly as in the initial position of the device discussed in the state of the art. At this point, the translation initiates up to when the first reflecting surface 8 reaches the first equilibrium distance (equal optical paths) generating, through a ray reflected by the beam 20, the first interference peak 30 (see figure 3).

As discussed in the state of the art, the temporal tracing of the signal obtained by the interferometer during the translation of the reference plates contains therefore a series of maxima, in correspondence of all the axial positions of the intraocular surfaces to measure. In particular, there is the first intense maximum precisely in correspondence of reaching the first plate 8 in correspondence of the anterior surface of the cornea. All the maxima are surrounded by an envelope having an oscillating shape and caused by secondary minima and maxima of the interference, due to the partial coherence of the light emitted by the SLED. Known algorithms of envelope therefore allow the extraction of the maximum.

The plates are made of untreated glass, reflecting in a range variable between 1% and about 4%, preferably 4%. In this way, the plates are not darkening and the light passes and is reflected also by the posterior plate 9. The system would not work with mirrors or high-reflectance plates since in that case the posterior plate would not be hit by any beam, which is instead totally shielded by the anterior plate.

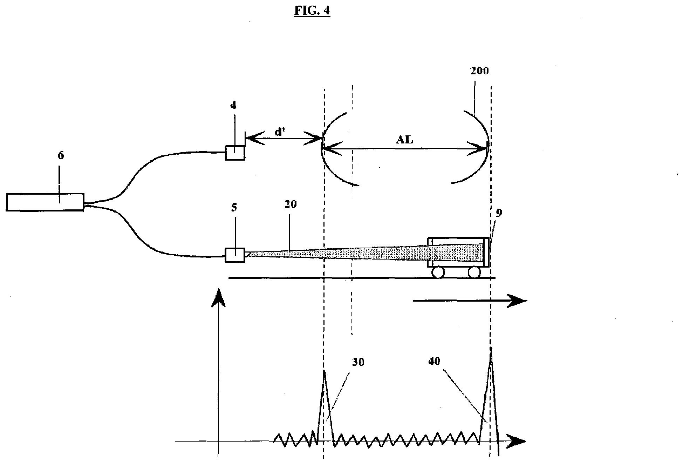

Going on with the translation the cylinder reaches the surface of the retina 200 (see figure 4). In particular, the second plate 9, when it reaches an optical path equivalent to that of the retina, reflects the beam 20, generating the second interference peak 40 (see figure 4). The 4% of energy that returns from each plate is more than enough to make the measuring. Obviously, the second surface 9 is hit by the 96% of the radiation that affects the first one 8 (if the first plate is reflecting at 4%).

Thanks to the fact that the cylinder has a pre-determined geometrical length L the second reflecting surface will intercept the retina (that is the distance of equilibrium in which the optical paths are equal) much earlier with respect to the background art, that is the two peaks will be much nearer with each other.

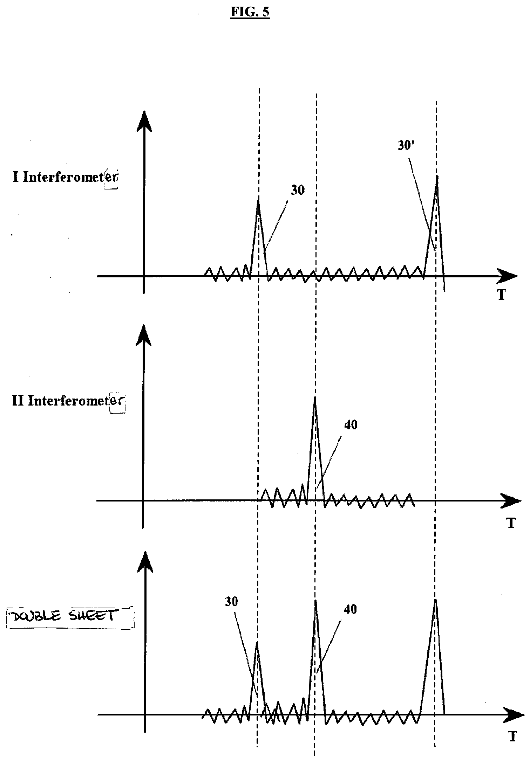

For that purpose, figure 5 clarifies it since it assimilates said system with two interferometers that work independently. It is therefore highlighted that the first plate 8, once the cornea is intercepted, generates the peak 30. The peak 30' is the one that is generated when the same plate reaches the optical distance corresponding to the position of a second reflecting ocular surface, for example the retina. In this case, as per the background art, the time T required for the measuring would be long.

The second interferometer, however, has its own plate 9 placed at an optical path near the retina. Therefore, it generates almost immediately after the generation of the first peak 30, the second peak 40. The double plate or slab, in accordance with the invention, therefore generates a signal that provides the two peaks 30 and 40 very near to each other and therefore obtained in a halved **T** time with respect to a system of the background art.

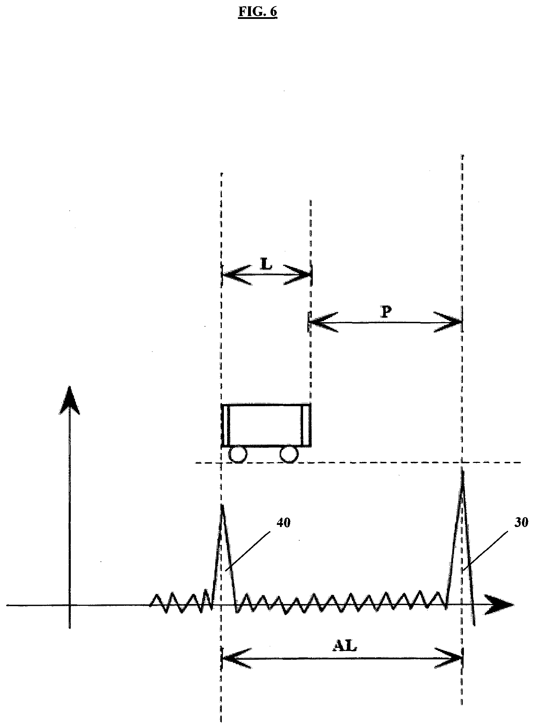

In this way, as schematically shown in figure 6, the distance AL sought is now easily implemented with a calculation that takes advantage of the distance P covered by the cylinder, distance in which the cylinder 7 has generated the two peaks (30, 40), added to the length L of the cylinder (this multiplied appropriately by the index of refraction of the cylinder glass) and the whole divided by the index of refraction of the eye.

It is not necessary, therefore, as described in the background art, to carry out a high-frequency oscillation, or every time to adjust the distances between the two plates to the one that is the distance that is expected. Starting from an initial position a single run in one direction is enough to have a tracing that allows to obtain the desired measure.

It is then in case possible to repeat the single run with a second or more runs (for example three) which possibly serve, but not necessarily, to obtain a verification tracing. It is obvious that, nevertheless, it is not a high-frequency oscillation in which innumerable runs per minute are made, forwards and backwards, at a precise temporal cadence.

As per figure 1, the PC controls the movement of the cylinder by means of a guide engine 500. In particular, the PC memorizes initial and final positions of movement, always commanding such a translation between these two extreme points each time an optical measuring is commanded.

An important aspect of the present invention, as schematically shown in figure 8, concerns the initial positioning of the Michelson interferometer with respect to the eye 500. To that aim, interferometer 1 (schematized in figure) is assembled on a structure 200 that provides a manual or automatic translation system 210 (double direction of the arrow). On the structure 200 the entire interferometer 1 and an apparatus of acquisition of the image (300, 400) are placed. There exist and have long been known on ophthalmological equipments apparatuses for the acquisition of images since they are used to reconstruct shapes of optical surfaces. In this case, the acquisition of the image is used in an innovative way.

The acquisition of the image can be carried out in various different ways. For example, in a case, through the arrangement of luminous LEDs directly on the Placido disk 300. The LEDs thus project the light on the ocular cornea.

Alternatively, a luminous "pattern" can be used obtained with appropriate LEDs positioned in different points from the Placido disk and that also project a luminous "pattern" on the surface of which the image wants to be acquired.

A videocamera 400 acquires the images that are analyzed through an appropriate software.

The translation system 210 is motorized and is controlled manually or automatically in such a way as to cause ad advancement and a retrocession of the entire structure 200 on which the interferometer 1 in arranged with respect to the surface 500 to acquire, thus acquiring various images (for example 25 frames per second) at different focal distances (Fd). Such images are analyzed with known software techniques to find the optimal focus distance (Fdc). By optimal focus distance it is intended the distance at which the videocamera takes an image perfectly in focus. Analysis algorithms to evaluate the quality of focus of an image are already known and therefore will not be further described in detail here.

Therefore, in a manual way the user can move in a direction or in the opposite direction the structure 200 until the position in which the software indicates that the optimal focus position has been reached, or, alternatively, this can take place automatically.

In accordance with the invention, therefore, the interferometer 1 is arranged on the structure and therefore translates integrally to the structure 200 during the search of the image in focus. In particular, the interferometer is placed in such a way so that when the image results in focus it is at a distance in which the optical path of the light reflected by the cornea 100 along the branch 4' is equal to the optical path of the light reflected from the first plate 8 in the branch 5' (equal optical distances d' and d"). Basically, when the image is in focus the distance of the interferometer from the cornea is substantially such as to generate immediately the first interference peak.

It is reminded that the frame 200 provides indeed a support base for the chin of the patient, so that the ocular distance from the videocamera and from the interferometer can easily be adjusted as said above.

This adjustment of initial position of the interferometer, which takes advantage of an acquisition of an image in focus, has the advantage of speeding up the process of measuring of the distance, rendering it precise above all.

First of all, any ambiguity is eliminated with respect to the background art since the first peak that is detected is certainly that of cornea 100. All the other peaks are then surfaces internal to the eye with respect to which the distance wants to be measured. The translation run becomes in this way very efficient since a single run allows to measure also more distances of different surfaces and it is not necessary anymore each time to have to adjust the two plates at a distance that is the one that is expected to be measured. It is enough to adjust or select two plates at a single pre-chosen distance of a value inferior to the measurings to be carried out and the same plates, with a single run, can be used from the starting point to scan all the surfaces of interest with a single run.

Basically, being the interferometer positioned in a known initial condition in which the first interference peak is immediately found, then the entire run can be taken advantage of to find other ocular surfaces, thus increasing the range of intraocular axial measuring.

In the present description the term luminous beam indicates in a totally generic and not limiting manner a beam that, as said, is generally in the field of the infrared.

## Claims

1. A Michelson-type interferometer (1) for measuring the intraocular axial length (AL) and comprising:
- A coupler (6) to which the four branches of optical fiber converge (2', 3', 4', 5'), a first branch (2') being connected to a light source (2) and a second branch (3') being connected to the receiving photodiode (3) that elaborates in return a reflected light beam, on the opposite part to the coupler (6) two branches of optical fiber (4', 5') converge and of which the branch (4') connected to a first collimator (4) in use destined to be placed opposite an eye (100) to be measured and the other one branch (5') connected to a second collimator (5) placed opposite to a reflecting arrangement (7), so that, in use, the beam emitted by the source (2) can be sent in part to the eye (100) and in part to the reflecting arrangement (7) through the two ramifications (4', 5') in such a way that the receiving photodiode (3), in return, can receive the beam reflected through the branch (3') and can analyze the interference between the reflected waves;
- The said reflecting arrangement (7) being at least partially reflecting the light and being translatable along a motion direction (P) so as to be able to move apart and to come near with respect to the second collimator (5), said arrangement (7) comprising a first surface (8) and a second surface (9), both said two surfaces (8, 9) being at least partially reflecting and arranged with each other at a pre-determined reciprocal distance (d);
- Said two reflecting surfaces (8, 9) being arranged in the arrangement (7) so as to keep constant said reciprocal distance (d) at least during the translation of the arrangement (7) in the measurement phase;
- A motorized driving system (500) to command the movement of the reflecting arrangement (7);
- said motorized driving system (500) being adapted to be controlled in such a way that the measurement can be completed in a single translation run in a direction starting from an initial position in which the first reflecting surface (8) generates the first interference peak at the beginning of the translation and with such a fixed distance (d) between the said two reflecting surfaces (8, 9) that afterwards during said translation the second reflecting surface (9) generates the second interference peak;
- **Characterized in that:**
- The reflecting arrangement (7) has the shape of a cylinder which anterior face provides the first reflecting surface (8), while the posterior face constitutes the second reflecting surface (9), which are coaxial and parallel with each other, said two reflecting surfaces (8, 9) being fixed
- The distance between the first reflecting surface (8) and the second reflecting surface (9) is comprised between 12mm and 19mm;
- said first (8) and second surface (9) are made of untreated glass, reflecting in a range variable between 1% and about 4%,
- said reflecting arrangement (7) has an optical length comprised between 15mm and 25mm.

2. An interferometer, as per claim 1, wherein the motorized system (500) is connected to an electronic processor (PC) that commands the motion within two extreme positions.

3. An interferometer (1), as per one or more of the preceding claims, wherein the two reflecting surfaces (8, 9) are of low reflectance in such a way as to reflect 4% of the beam that hits them.

4. An interferometer (1), as per one or more of the preceding claims, wherein the following are further provided:
- A system of acquisition/analysis of the images (300, 400) configured to acquire a plurality of images at different focal distances and to select the image that results in focus;
- A translation system (210) that allows to translate the interferometer (1) as a function of the images acquired in such a way that when the image acquired results in focus the interferometer results positioned with the first reflecting surface (8) substantially at an interference distance with respect to the external surface (100) of the cornea.

5. An interferometer (1), as per claim 4, wherein a frame is provided on which said acquisition/analysis system of the images and the interferometer (1) are arranged, said frame being reciprocable in such a way as to allow the acquisition of images at different focal distances.

6. A method for measuring the intraocular axial length (AL) by means of a Michelson-type interferometer (1) accordingly to one or more of the previous claims, said method comprising the following operations:
- Initial positioning of the reflecting arrangement (7) in such a way that the first reflecting surface (8) is found at a distance (d") from the second collimator (5) inferior to the distance (d') of the first collimator (4) from the external surface of the cornea (100);
- Sending of the light beam through the two branches (4', 5') terminating in the two collimators (4, 5);
- Translation of the reflecting arrangement (7) by operating a single translation run along a measurement direction in such a way as to obtain the generation of two interference peaks (30, 40) in correspondence of the reaching of the two interference positions in which respectively the optical distance of the first reflecting surface (8) from the second collimator (5) is equal to the optical distance of the external surface of the cornea (100) from the first collimator (4) and the optical distance of the second reflecting surface (9) from the second collimator (5) is equal to the optical distance of the first collimator (4) from the retina;
- And wherein the said two reflecting surfaces (8, 9) are arranged in the arrangement (7) in such a way as to keep constant said reciprocal distance (d) at least during the translation of the arrangement (7) in the measurement phase and such that the first reflecting surface (8) generates the first interference peak at the beginning of the translation and afterwards during said translation the second reflecting surface (9) generates the second interference peak.

7. A method, as per claim 6, wherein an operation of acquisition of a plurality of images at different focal distances is preliminarily provided with a consequent translation of the interferometer as a function of the optimal focal distance obtained such that when the image acquired results in focus the interferometer results positioned at a distance in which the first reflecting surface (8) substantially generates immediately interference with respect to the external surface (100) of the cornea.

## Patentansprüche

1. Interferometer vom Michelson-Typ (1) zur Messung der intraokularen axialen Länge (AL), umfassend:
- Einen Koppler (6), zu dem die vier Zweige der optischen Faser konvergieren (2', 3', 4', 5'), wobei ein erster Zweig (2') mit einer Lichtquelle (2) verbunden ist und ein zweiter Zweig (3') mit der empfangenden Fotodiode (3) verbunden ist, die wiederum einen reflektierten Lichtstrahl ausarbeitet, auf dem gegenüberliegenden Teil des Kopplers (6) zwei Zweige der optischen Faser (4', 5') konvergieren und von denen der Zweig (4') mit einem ersten Kollimator (4) im Gebrauch verbunden ist, der dazu bestimmt ist, einem zu messenden Auge (100) gegenübergestellt zu werden, und der andere Zweig (5') mit einem zweiten Kollimator (5) verbunden ist, der einer reflektierenden Anordnung (7) gegenübergestellt ist, so dass im Gebrauch der von der Quelle (2) emittierte Strahl teilweise zum Auge (100) und teilweise zur reflektierenden Anordnung (7) durch die zwei Verzweigungen (4', 5') geschickt werden kann, so dass die empfangende Fotodiode (3) wiederum den durch den Zweig (3') reflektierten Strahl empfangen und die Interferenz zwischen den reflektierten Wellen analysieren kann;
- Wobei die reflektierende Anordnung (7) das Licht zumindest teilweise reflektiert und entlang einer Bewegungsrichtung (P) translatorisch verschiebbar ist, um sich in Bezug auf den zweiten Kollimator (5) auseinander bewegen und annähern zu können, wobei die Anordnung (7) eine erste Oberfläche (8) und eine zweite Oberfläche (9) umfasst, wobei beide zwei Oberflächen (8, 9) zumindest teilweise reflektierend sind und miteinander in einem vorbestimmten gegenseitigen Abstand (d) angeordnet sind;
- Die zwei reflektierenden Oberflächen (8, 9) sind in der Anordnung (7) so angeordnet, dass der gegenseitige Abstand (d) zumindest während der Translation der Anordnung (7) in der Messphase konstant gehalten wird;
- Ein motorisiertes Antriebssystem (500), um die Bewegung der reflektierenden Anordnung (7) zu befehlen;
- wobei das motorisierte Antriebssystem (500) so gesteuert werden kann, dass die Messung in einem einzigen Translationslauf in einer Richtung ausgehend von einer Ausgangsposition, in der die erste reflektierende Oberfläche (8) den ersten Interferenzpeak zu Beginn der Translation erzeugt, und mit einem solchen festen Abstand (d) zwischen den zwei reflektierenden Oberflächen (8, 9), dass danach während der Translation die zweite reflektierende Oberfläche (9) den zweiten Interferenzpeak erzeugt, abgeschlossen werden kann;
- **Dadurch gekennzeichnet, dass:**
- Die reflektierende Anordnung (7) die Form eines Zylinders aufweist, wobei die vordere Fläche die erste reflektierende Oberfläche (8) bereitstellt, während die hintere Fläche die zweite reflektierende Oberfläche (9) bildet, die koaxial und parallel zueinander sind, wobei die zwei reflektierenden Oberflächen (8, 9) feststehend sind,
- Der Abstand zwischen der ersten reflektierenden Oberfläche (8) und der zweiten reflektierenden Oberfläche (9) zwischen 12 mm und 19 mm beträgt;
- die erste (8) und die zweite Oberfläche (9) aus unbehandeltem Glas bestehen und in einem Bereich reflektieren, der zwischen 1 % und etwa 4 % variabel ist,
- die reflektierende Anordnung (7) eine optische Länge zwischen 15 mm und 25 mm aufweist.

2. Interferometer nach Anspruch 1, wobei das motorisierte System (500) mit einem elektronischen Prozessor (PC) verbunden ist, der die Bewegung innerhalb von zwei Extrempositionen befiehlt.

3. Interferometer (1) nach einem oder mehreren der vorhergehenden Ansprüche, wobei die zwei reflektierenden Oberflächen (8, 9) ein geringes Reflexionsvermögen aufweisen, um 4 % des auf sie auftreffenden Strahls zu reflektieren.

4. Interferometer (1) nach einem oder mehreren der vorhergehenden Ansprüche, wobei ferner Folgendes vorgesehen ist:
- Ein System zur Erfassung/Analyse der Bilder (300, 400), das dazu konfiguriert ist, eine Vielzahl von Bildern mit unterschiedlichen Brennweiten zu erfassen und das Bild auszuwählen, das zu einer Fokussierung führt;
- Ein Translationssystem (210), das es ermöglicht, das Interferometer (1) in Abhängigkeit von den erfassten Bildern so zu translatieren, dass, wenn das erfasste Bild zu einer Fokussierung führt, das Interferometer mit der ersten reflektierenden Oberfläche (8) im Wesentlichen in einem Interferenzabstand in Bezug auf die äußere Oberfläche (100) der Hornhaut positioniert wird.

5. Interferometer (1) nach Anspruch 4, wobei ein Rahmen vorgesehen ist, auf dem das Erfassungs-/Analysesystem der Bilder und das Interferometer (1) angeordnet sind, wobei der Rahmen so hin- und herbewegbar ist, dass er die Erfassung von Bildern mit unterschiedlichen Brennweiten ermöglicht.

6. Verfahren zur Messung der intraokularen axialen Länge (AL) mittels eines Interferometers vom Michelson-Typ (1) nach einem oder mehreren der vorhergehenden Ansprüche, wobei das Verfahren die folgenden Vorgänge umfasst:
- Anfängliche Positionierung der reflektierenden Anordnung (7) derart, dass sich die erste reflektierende Oberfläche (8) in einem Abstand (d") vom zweiten Kollimator (5) befindet, der geringer ist als der Abstand (d') des ersten Kollimators (4) von der äußeren Oberfläche der Hornhaut (100);
- Senden des Lichtstrahls durch die zwei Zweige (4', 5'), die in den zwei Kollimatoren (4, 5) enden;
- Translation der reflektierenden Anordnung (7) durch Betreiben eines einzigen Translationslaufs entlang einer Messrichtung, um die Erzeugung von zwei Interferenzpeaks (30, 40) entsprechend dem Erreichen der zwei Interferenzpositionen zu erhalten, in denen jeweils der optische Abstand der ersten reflektierenden Oberfläche (8) vom zweiten Kollimator (5) gleich dem optischen Abstand der äußeren Oberfläche der Hornhaut (100) vom ersten Kollimator (4) ist und der optische Abstand der zweiten reflektierenden Oberfläche (9) vom zweiten Kollimator (5) gleich dem optischen Abstand des ersten Kollimators (4) von der Netzhaut ist;
- Und wobei die zwei reflektierenden Oberflächen (8, 9) derart in der Anordnung (7) angeordnet sind, dass der gegenseitige Abstand (d) zumindest während der Translation der Anordnung (7) in der Messphase konstant gehalten wird und dass die erste reflektierende Oberfläche (8) zu Beginn der Translation den ersten Interferenzpeak und danach während der Translation die zweite reflektierende Oberfläche (9) den zweiten Interferenzpeak erzeugt.

7. Verfahren nach Anspruch 6, wobei ein Vorgang der Erfassung einer Vielzahl von Bildern bei unterschiedlichen Brennweiten vorläufig mit einer konsequenten Translation des Interferometers als Funktion der optimalen Brennweite vorgesehen wird, die erhalten wird, so dass, wenn das erfasste Bild zu einer Fokussierung führt, das Interferometer in einem Abstand positioniert wird, in dem die erste reflektierende Oberfläche (8) im Wesentlichen eine sofortige Interferenz in Bezug auf die äußere Oberfläche (100) der Hornhaut erzeugt.

## Revendications

1. Interféromètre de type Michelson (1) pour la mesure de la longueur axiale (AL) intraoculaire et comprenant :
- un coupleur (6) vers lequel les quatre branches d'une fibre optique convergent (2', 3', 4', 5'), une première branche (2') étant reliée à une source de lumière (2) et une seconde branche (3') étant reliée à la photodiode réceptrice (3) qui élabore en retour un faisceau de lumière réfléchi, sur la partie opposée au coupleur (6) deux branches de fibre optique (4', 5') convergent et parmi lesquelles la branche (4') reliée à un premier collimateur (4) en utilisation destiné à être placé à l'opposé d'un œil (100) devant être mesuré et l'autre branche (5') reliée à un second collimateur (5) placé à l'opposé d'un agencement réfléchissant (7), de sorte que, en utilisation, le faisceau émis par la source (2) puisse être envoyé en partie vers l'œil (100) et en partie vers l'agencement réfléchissant (7) à travers les deux ramifications (4', 5') d'une manière telle que la photodiode réceptrice (3), en retour, puisse recevoir le faisceau réfléchi à travers la branche (3') et puisse analyser l'interférence entre les ondes réfléchies ;
- ledit agencement réfléchissant (7) réfléchissant au moins partiellement la lumière et étant apte à être déplacé en translation le long d'une direction de mouvement (P) de façon à être apte à s'éloigner et se rapprocher par rapport au second collimateur (5), ledit agencement (7) comprenant une première surface (8) et une seconde surface (9), lesdites deux surfaces (8, 9) étant au moins partiellement réfléchissantes et agencées l'une par rapport à l'autre à une distance réciproque (d) prédéterminée ;
- lesdites deux surfaces réfléchissantes (8, 9) étant agencées dans l'agencement (7) de façon à maintenir constante ladite distance réciproque (d) au moins pendant le déplacement en translation de l'agencement (7) dans la phase de mesure ;
- un système d'entraînement motorisé (500) pour commander le mouvement de l'agencement réfléchissant (7) ;
- ledit système d'entraînement motorisé (500) étant conçu pour être commandé d'une manière telle que la mesure puisse être achevée en une seule course de déplacement en translation dans une direction commençant à partir d'une position initiale dans laquelle la première surface réfléchissante (8) génère le premier pic d'interférence au début du déplacement en translation et avec une distance fixe (d) entre lesdites deux surfaces réfléchissantes (8, 9) qui est telle que par la suite, pendant ledit déplacement en translation, la seconde surface réfléchissante (9) génère le second pic d'interférence ;
- **caractérisé en ce que** :
- l'agencement réfléchissant (7) possède la forme d'un cylindre dont la face antérieure fournit la première surface réfléchissante (8), tandis que la face postérieure constitue la seconde surface réfléchissante (9), lesquelles sont coaxiales et parallèles l'une à l'autre, lesdites deux surfaces réfléchissantes (8, 9) étant fixes,
- la distance entre la première surface réfléchissante (8) et la seconde surface réfléchissante (9) est comprise entre 12 mm et 19 mm ;
- ladite première (8) et ladite seconde surface (9) sont constituées de verre non traité, réfléchissant dans une plage variable entre 1 % et environ 4 %,
- ledit agencement réfléchissant (7) possède une longueur optique comprise entre 15 mm et 25 mm.

2. Interféromètre selon la revendication 1, dans lequel le système motorisé (500) est connecté à un processeur électronique (PC) qui commande le mouvement dans les limites de deux positions extrêmes.

3. Interféromètre (1) selon l'une ou plusieurs des revendications précédentes, dans lequel les deux surfaces réfléchissantes (8, 9) possèdent une faible réflectance de manière à réfléchir 4 % du faisceau qui les atteint.

4. Interféromètre (1) selon l'une ou plusieurs des revendications précédentes, dans lequel les éléments suivants sont en outre fournis :
- un système d'acquisition/d'analyse des images (300, 400) configuré pour acquérir une pluralité d'images à différentes distances focales et pour sélectionner l'image qui se retrouve mise au point ;
- un système de translation (210) qui permet de déplacer en translation l'interféromètre (1) en fonction des images acquises d'une manière telle que, lorsque l'image acquise se retrouve mise au point, l'interféromètre se retrouve positionné avec la première surface réfléchissante (8) sensiblement à une distance d'interférence par rapport à la surface externe (100) de la cornée.

5. Interféromètre (1) selon la revendication 4, dans lequel un cadre est ménagé sur lequel ledit système d'acquisition/d'analyse des images et l'interféromètre (1) sont agencés, ledit cadre étant apte à effectuer des va-et-vient de manière à permettre l'acquisition d'images à différentes distances focales.

6. Procédé de mesure de la longueur axiale (AL) intraoculaire au moyen d'un interféromètre de type Michelson (1) selon l'une ou plusieurs des revendications précédentes, ledit procédé comprenant les opérations suivantes :
- le positionnement initial de l'agencement réfléchissant (7) d'une manière telle que la première surface réfléchissante (8) se trouve à une distance (d") du second collimateur (5) inférieure à la distance (d') du premier collimateur (4) par rapport à la surface externe de la cornée (100) ;
- l'envoi du faisceau de lumière à travers les deux branches (4', 5') se terminant dans les deux collimateurs (4, 5) ;
- le déplacement en translation de l'agencement réfléchissant (7) par la réalisation d'une unique course de déplacement en translation le long d'une direction de mesure de manière à obtenir la génération de deux pics d'interférence (30, 40) en correspondance avec le fait d'atteindre les deux positions d'interférence dans lesquelles, respectivement, la distance optique de la première surface réfléchissante (8) par rapport au second collimateur (5) est égale à la distance optique de la surface externe de la cornée (100) par rapport au premier collimateur (4) et la distance optique de la seconde surface réfléchissante (9) par rapport au second collimateur (5) est égale à la distance optique du premier collimateur (4) par rapport à la rétine ;
- et dans lequel lesdites deux surfaces réfléchissantes (8, 9) sont agencées dans l'agencement (7) de manière à maintenir constante ladite distance réciproque (d) au moins pendant le déplacement en translation de l'agencement (7) dans la phase de mesure et de sorte que la première surface réfléchissante (8) génère le premier pic d'interférence au début du déplacement en translation et par la suite, pendant ledit déplacement en translation, la seconde surface réfléchissante (9) génère le second pic d'interférence.

7. Procédé selon la revendication 6, dans lequel une opération d'acquisition d'une pluralité d'images à différentes distances focales est effectuée de manière préliminaire avec un déplacement en translation consécutif de l'interféromètre en fonction de la distance focale optimale obtenue de telle sorte que, lorsque l'image acquise se retrouve mise au point, l'interféromètre se retrouve positionné à une distance à laquelle la première surface réfléchissante (8) génère sensiblement immédiatement une interférence par rapport à la surface externe (100) de la cornée.
